# EUROPÄISCHE PATENTSCHRIFT

(11) **EP 1 987 790 B1**
(45) Veröffentlichungstag und Bekanntmachung des Hinweises auf die Patenterteilung: **13.07.2016**
(21) Anmeldenummer: 08008152.4
(22) Anmeldetag: 29.04.2008
(51) Int. Cl.: A61B 17/16, A61B 17/00, A61B 17/29

(54) **Zerlegbares medizinisches Zangensystem**
Dismountable medical pincer system
Système de pinces médicales démontables

(30) Priorität: 04.05.2007 DE 102007021658
(43) Veröffentlichungstag der Anmeldung: 05.11.2008
(60) Teilanmeldung: 10185212.7
(73) Patentinhaber: Karl Storz GmbH & Co. KG, 78532 Tuttlingen (DE)
(72) Erfinder: Stefan, Jochen, 88639 Wald (DE); Bacher, Uwe, 78532 Tuttlingen (DE)
(74) Vertreter: Witte, Weller & Partner Patentanwälte mbB

(56) Entgegenhaltungen:
- WO-A-92/14414
- DE-A1- 10 038 085
- US-A- 5 282 800
- US-A- 6 099 537
- US-A1- 2004 230 221
- US-A1- 2005 165 443

## Beschreibung

Die Erfindung betrifft ein zerlegbares medizinisches Zangensystem, mit einem ersten Kraftübertragungselement, das zur Kraftübertragung einer ersten maximalen Kraft ausgelegt ist, mit zumindest einem zweiten Kraftübertragungselement, das zur Kraftübertragung einer zweiten maximalen Kraft ausgelegt ist, die größer ist als die erste maximale Kraft, und mit zumindest einem ersten Griff, der zumindest ein bewegliches Griffteil aufweist, wobei der zumindest eine erste Griff eine Kupplungsvorrichtung aufweist, mittels der eines der Kraftübertragungselemente kraftschlüssig mit dem beweglichen Griffteil verbunden werden kann, wobei das erste Kraftübertragungselement ein erstes Verbindungselement und das zumindest zweite Kraftübertragungselement ein zweites Verbindungselement zur jeweiligen Verbindung der Kupplungsvorrichtung aufweist, wobei die Kupplungsvorrichtung eine erste Aufnahme, die zur kraftschlüssigen Verbindung mit dem ersten Verbindungselement ausgelegt ist, und zumindest eine zweite Aufnahme aufweist, die zur kraftschlüssigen Verbindung mit dem zweiten Verbindungselement ausgelegt ist.

Ein solches zerlegbares medizinisches Zangensystem ist aus dem Dokument US 2005/0165443 A1 bekannt.

Ein zerlegbares medizinisches Zangensystem ist unter der Marke CLICKline® bekannt, das von der Karl Storz GmbH & Co. KG vertrieben wird und beispielsweise in dem Firmenkatalog "Storz Karl Storz-Endoskope, Band Laparoskopie, 5. Ausgabe, 2005", Seiten 72ff. beschrieben ist.

Medizinische Zangen werden in der offenen oder in der endoskopischen Chirurgie beispielsweise zum Schneiden, Fassen, Koagulieren, usw. von Gewebe im menschlichen oder tierischen Körper verwendet.

Eine medizinische Zange weist allgemein einen Griff mit zumindest einem beweglichen Griffteil, einen lang erstreckten Schaft, der mit dem Griff verbunden ist, zumindest ein bewegliches Werkzeug, beispielsweise Maulteil, am distalen Ende des Schafts sowie ein Kraftübertragungselement auf, üblicherweise in Form einer Zug- und Druckstange, das das zumindest eine bewegliche Werkzeug mit dem zumindest einen beweglichen Griffteil des Griffs kraftschlüssig verbindet. Durch Bewegen des zumindest einen beweglichen Griffteils wird die dabei erzeugte Kraft von dem Kraftübertragungselement auf das zumindest eine beweglichen Werkzeug zum Bewegen desselben übertragen.

Aus Gründen der besseren Reinigbarkeit werden solche medizinischen Zangen zerlegbar ausgestaltet, wobei sich eine zerlegbare Zange üblicherweise in die Baugruppen Griff, Schaft und Kraftübertragungselement zerlegen lässt, wobei mit dem Kraftübertragungselement auch das zumindest eine Werkzeug als Baueinheit verbunden sein kann, die auch als Arbeitseinsatz der Zange bezeichnet wird.

Die Zerlegbarkeit einer medizinischen Zange erhöht nicht nur die Reinigungsfreundlichkeit der Zange, sondern erhöht auch die Funktionsvariabilität einer solchen Zange, indem verschiedene Griffe mit verschiedenen Schäften und/oder Arbeitseinsätzen kombiniert werden können.

Das bekannte Zangensystem umfasst einerseits mehrere verschiedene Griffe und andererseits eine Mehrzahl an verschiedenen Schäften und Arbeitseinsätzen. So können die Griffe sich hinsichtlich der jeweils erzeugbaren maximalen Kraft unterscheiden, indem beispielsweise Griffe bereitgestellt werden, deren zumindest eines bewegliches Griffteil aufgrund hoher Hebelwirkung eine große Kraft erzeugen kann, während andere Griffe kleiner ausgebildet sind und eine entsprechend kleinere Kraft erzeugen. Eine höhere Kraft ist beispielsweise zum Schneiden bzw. Stanzen von Knochengewebe erforderlich, während zum feinen Präparieren von weicherem Gewebe ein kleinerer Handgriff besser geeignet ist, weil mit ihm die von der Hand des Arztes ausgeübte Kraft besser dosierbar ist.

Auch die Kraftübertragungselemente können sich hinsichtlich der mit ihnen maximal übertragbaren Kraft unterscheiden. Die mit dem jeweiligen Kraftübertragungselement maximal übertragbare Kraft ist durch die Dicke bzw. Stärke des Kraftübertragungselements und seines Verbindungselements zur Verbindung mit dem beweglichen Griffteil begrenzt. Zum Schneiden von Knochengewebe muss von dem Griff eine hohe Kraft von dem Kraftübertragungselement auf das zumindest eine Werkzeug am distalen Ende übertragen werden, so dass ein Kraftübertragungselement für diese Zwecke mit einer gewissen Stärke ausgebildet sein muss, beispielsweise einem Durchmesser von 3 mm. Dies bedingt auch einen entsprechend größeren Außendurchmesser des Außenschafts, durch den sich das Kraftübertragungselement zwischen dem Griff und dem zumindest einen Werkzeug erstreckt.

In räumlich engen Operationsgebieten, wie beispielsweise im Bereich der HNO, sind dagegen sehr schlank bauende Außenschäfte mit geringem Durchmesser erforderlich, so dass das Kraftübertragungselement entsprechend ebenfalls relativ dünn ausgebildet werden muss, beispielsweise mit einem Durchmesser von etwa 1 mm.

Wünschenswert ist es, um eine möglichst hohe Kompatibilität zu erreichen, dass Kraftübertragungselemente, die für die verschiedenen maximal übertragbaren Kräfte ausgelegt sind, mit ein und demselben Griff verbinden werden können, um die Anzahl an bereitzustellenden Griffen möglichst gering zu halten.

Das oben genannte bekannte zerlegbare Zangensystem ist jedoch nicht in der Lage, Kraftübertragungselemente mit unterschiedlichen Durchmessern in einem Griff aufzunehmen. Vielmehr sind die Verbindungselemente der Kraftübertragungselemente einerseits und der Kupplungsvorrichtung des oder der Griffe einheitlich ausgebildet, damit möglichst jedes Kraftübertragungselement mit ein und demselben Griff verbunden werden kann. Dies führt jedoch zu den nachstehend erläuterten technischen Problemen, insbesondere wenn Kraftübertragungselemente mit stark unterschiedlichen Stärken und damit Beanspruchbarkeiten in einem Zangensystem zusammengefasst werden sollen.

Bei der Vormontage des Arbeitseinssatzes wird das Kraftübertragungselement von distal her durch den Außenschaft geschoben. Das Verbindungselement am proximalen Ende des Kraftübertragungselements, das beispielsweise in Form einer Kugel ausgebildet ist, muss dabei ebenfalls durch den Außenschaft treten können, bis es aus dem proximalen Ende des Außenschafts vorragt. Ist das Kraftübertragungselement nun auf eine große Kraftübertragung ausgelegt, muss auch das Verbindungselement mit entsprechender Stärke ausgelegt sein, damit die Verbindung zwischen dem Kraftübertragungselement und dem Griff insgesamt großen Kräften standhalten kann. Damit das relativ groß dimensionierte Verbindungselement durch den Außenschaft hindurchtreten kann, muss der Außenschaft einen relativ großen Durchmesser aufweisen. Ein solches Zangensystem hat dann den Nachteil, dass bei einer Verbindung des Griffs mit einem dünneren Kraftübertragungselement der Außenschaft dennoch den gleichen großen Durchmesser aufweisen muss wie im Fall der Verbindung eines stärker dimensionierten Kraftübertragungselements mit dem Griff, damit die Kompatibilität zwischen verschiedenen Kraftübertragungselementen und demselben Griff nicht aufgegeben werden muss. Andernfalls müsste ein zweiter Griff mit einer anderen Kupplungsvorrichtung bereitgestellt werden, was jedoch dem Sinn einer höheren Kompatibilität zuwiderläuft und die Anzahl an unterschiedlichen Griffen unerwünscht erhöht.

Wird umgekehrt die Verbindung zwischen dem Kraftübertragungselement und dem Griff auf das Kraftübertragungselement mit kleiner maximaler Kraftübertragung ausgelegt, so dass das Verbindungselement am proximalen Ende des Kraftübertragungselements kleiner dimensioniert werden kann, kann zwar die Außenschaftabmessung variabel und insbesondere dünn gewählt werden, die maximal übertragbare Kraft bleibt aber auf diejenige des kleinsten bzw. schwächsten Kraftübertragungselements begrenzt. Dies bedeutet, dass beim Einsatz von viel Kraft erfordernden Werkzeugen, wie beispielsweise Knochenstanzen, mit einem entsprechend groß dimensionierten Griff, ein Bruch des Kraftübertragungselements im Bereich des Verbindungselements nicht ausgeschlossen werden kann, wenn der Griff mit großer Handkraft betätigt wird.

Der die Zange betätigende Arzt muss daher in diesem Fall seine Handkraft behutsam dosieren, um einen Bruch des Kraftübertragungselements zu vermeiden.

Das bekannte medizinische Zangensystem hat daher den Nachteil, dass die Verbindungselemente der vorhandenen Kraftübertragungselemente auf nur eine Stärkendimensionierung festgelegt sind, was entweder zu unerwünscht großen Außendurchmessern der Außenschäfte oder der Gefahr eines Bruches des Kraftübertragungselements führt.

Das aus dem eingangs genannten Dokument US 2005/0165443 A1 bekannte zerlegbare Zangensystem weist einen Handgriff und zumindest zwei Arbeitseinsätze auf, von denen der eine unter Wirkung von monopolarem Hochfrequenzstrom und der andere unter Wirkung von bipolarem Hochfrequenzstrom arbeitet, um Gewebe zu schneiden oder zu koagulieren. Jeder der beiden Arbeitseinsätze weist ein Kraftübertragungselement auf, das ein Verbindungselement zum kraftschlüssigen Verbinden des jeweiligen Kraftübertragungselements mit dem beweglichen Griffteil des Handgriffs ausgelegt ist. Das Verbindungselement des Kraftübertragungselements des einen Arbeitseinsatzes befindet sich an einer axial anderen Stelle als das Verbindungselement des Kraftübertragungselements des anderen Arbeiteinsatzes, und entsprechend sind an der Kupplungsvorrichtung des Handgriffs zwei voneinander axial beabstandete Aufnahmen für das jeweilige Verbindungselement vorhanden. Die Verbindungselemente der unterschiedlichen Kraftübertragungselemente unterscheiden sich im Querschnitt quer zur Längsrichtung des jeweiligen Kraftübertragungselements nicht voneinander, sondern weisen die gleiche Querschnittsgröße bzw. form quer zur Längsrichtung des jeweiligen Kraftübertragungselements auf.

Der Erfindung liegt die Aufgabe zugrunde, ein zerlegbares medizinisches Zangensystem der eingangs genannten Art dahingehend weiterzubilden, dass mit ein und demselben Griff Kraftübertragungselemente mit unterschiedlicher Kraftübertragungsauslegung verbunden werden können, ohne dass dadurch ein Mindestdurchmesser des Außenschaftes festgelegt ist.

Hinsichtlich des eingangs genannten zerlegbaren medizinischen Zangensystems wird die vorstehend genannte Aufgabe erfindungsgemäß dadurch gelöst, dass sich die Querschnittsgröße und/oder Querschnittsform des ersten Verbindungselements quer zur Längsrichtung des ersten Kraftübertragungselements von der Querschnittsgröße und/oder Querschnittsform des zweiten Verbindungselements quer zur Längsrichtung des zweiten Kraftübertragungselements unterscheidet.

Das erfindungsgemäße zerlegbare medizinische Zangensystem weist somit zumindest einen Griff auf, mit dem wahlweise zumindest zwei auf unterschiedliche maximale Kraftübertragung ausgelegte Kraftübertragungselemente verbunden werden können. Dazu ist die Kupplungsvorrichtung dieses ersten Griffs mit zumindest zwei Aufnahmen ausgestaltet, wobei die eine Aufnahme dazu ausgelegt ist, das Verbindungselement des ersten Kraftübertragungselements kraftschlüssig mit dem Griff zu verbinden, und die zumindest eine andere Aufnahme dazu ausgelegt ist, das Verbindungselement des zweiten Kraftübertragungselements kraftschlüssig mit demselben Griff zu verbinden. Bei dem erfindungsgemäßen zerlegbaren medizinischen Zangensystem kann somit die Anzahl an bereitzuhaltenden Griffen verringert werden, da ein und derselbe Griff mit unterschiedlichen Kraftübertragungselementen verbunden werden kann. Dies bedeutet weiterhin, dass ein für eine kleinere Kraftübertragung ausgelegtes Kraftübertragungselement, das entsprechend mit geringerer Stärke dimensioniert ist, zusammen mit einem schlankeren Schaft mit dem Griff verbunden werden kann, ebenso wie ein für eine höhere Kraftübertragung ausgelegtes stärker dimensioniertes Kraftübertragungselement, das mit einem entsprechend größer dimensionierten Schaft ebenso mit demselben Griff verbunden werden kann. Es versteht sich, dass die Kupplungsvorrichtung auch mehr als zwei Aufnahmen für mehr als zwei verschieden ausgelegte Kraftübertragungselemente aufweisen kann.

Der Begriff "Aufnahme" ist im Sinne der vorliegenden Erfindung allgemein zu verstehen und umfasst alle Ausgestaltungen derselben, die eine kraftschlüssige Verbindung des jeweiligen Verbindungselements mit der Aufnahme ermöglichen.

Im einfachsten Fall ist das erste Verbindungselement, wie in einer weiteren bevorzugten Ausgestaltung vorgesehen, kugelförmig mit einem ersten Durchmesser und das zweite Verbindungselement kugelförmig mit einem zweiten Durchmesser, der größer als der erste Durchmesser ist.

Die Ausgestaltung der Verbindungselemente in Kugelform hat den Vorteil einer einfacheren Herstellbarkeit, weil die Verbindungselemente als Drehteile und insbesondere in einem Arbeitsgang einstückig mit dem übrigen Körper des jeweiligen Kraftübertragungselements ausgebildet werden können. Außerdem ermöglicht es die kugelförmige Ausgestaltung der Verbindungselemente, dass sich diese in der jeweiligen Aufnahme trotz der kraftschlüssigen Verbindung um die Längsachse des Kraftübertragungselements drehen lassen, so dass sich das Werkzeug, beispielsweise ein oder mehrere Maulteile am distalen Ende des Instruments, relativ zum Griff drehen lassen, wodurch die Position des Werkzeugs ohne Veränderung der Position des Griffs von der Bedienungsperson verändert werden kann.

In einer weiteren bevorzugten Ausgestaltung des zerlegbaren medizinischen Zangensystems ist die erste Aufnahme von der zweiten Aufnahme axial beabstandet.

Hierbei ist von Vorteil, dass die erfindungsgemäß erreichte Möglichkeit, mit demselben Griff unterschiedlich ausgelegte Kraftübertragungselemente verbinden zu können, nicht zu Lasten einer höheren Querabmessung der Kupplungsvorrichtung und damit des Griffs im Bereich der Kupplungsvorrichtung geht, weil die zumindest zwei Aufnahmen der Kupplungsvorrichtung hintereinander in Längsrichtung der Kraftübertragungsrichtung angeordnet sind.

In diesem Zusammenhang ist bevorzugt, wenn das erste Verbindungselement durch die zweite Aufnahme hindurchgreift, wenn es in der ersten Aufnahme kraftschlüssig verbunden ist.

Diese Ausgestaltung eignet sich insbesondere dann, wenn das erste und zweite Verbindungselement hinsichtlich ihrer Querschnittsgröße unterschiedlich sind. Das Verbindungselement mit kleinerer Querschnittsgröße kann dann durch die distal gelegene zweite Aufnahme, die zur kraftschlüssigen Verbindung des Verbindungselements mit größerer Querschnittsgröße ausgelegt ist, ungehindert hindurchgreifen, um mit der ersten proximal gelegenen Aufnahme kraftschlüssig verbunden werden zu können. In dieser Ausgestaltung wird insbesondere der vorstehend genannte Vorteil einer sehr schlankbauenden Kupplungsvorrichtung noch besser verwirklicht.

In einer weiteren bevorzugten Ausgestaltung des Zangensystems weist die Kupplungsvorrichtung ein zwischen einer ersten Stellung und einer zweiten Stellung bewegliches Kupplungselement auf, das die erste und die zumindest zweite Aufnahme zumindest teilweise aufweist, wobei in der ersten Stellung das erste oder das zumindest zweite Verbindungselement in die erste oder zweite Aufnahme einführbar oder herausnehmbar ist, und in der zweiten Stellung das erste oder das zumindest zweite Verbindungselement in der ersten bzw. zweiten Aufnahme verriegelt ist.

Die Ausgestaltung der Kupplungsvorrichtung mit einem zwischen einer Einführstellung und einer Verriegelungsstellung beweglichen Kupplungselement hat den Vorteil, dass das kraftschlüssige Verbinden des jeweiligen Kraftübertragungselements mit dem Griff auf einfach handhabbare Weise erfolgen kann, und insbesondere, wenn das Kupplungselement mit einem Steuermechanismus versehen ist, vorzugsweise auf automatische Weise.

Dabei ist es bevorzugt, wenn das Kupplungselement durch eine Drehbewegung von der ersten Stellung in die zweite Stellung bzw. umgekehrt bewegbar ist, wobei das Kupplungselement mit einem axial beweglichen Schieber, der mit dem beweglichen Griffteil verbunden ist, verbunden ist und relativ zum Schieber axial unbeweglich ist, und wobei die Drehbewegung aus einer axialen Bewegung des Schiebers abgeleitet wird.

Durch die Mitnahme des Kupplungselements bei einer axialen Bewegung des Schiebers beim kraftschlüssigen Verbinden des ersten oder zweiten Verbindungselements mit dem Kupplungselement wird das Kupplungselement somit vorteilhafterweise selbsttätig von der Einführstellung in die Verriegelungsstellung gedreht und beim Entriegeln entsprechend umgekehrt.

In einer weiteren bevorzugten Ausgestaltung weist die erste Aufnahme einen Aufnahmeabschnitt, in dem das erste Verbindungselement im kraftschlüssig verbundenen Zustand zu liegen kommt, und einen Verriegelungsabschnitt auf, der einen Schlitz aufweist, der das Kraftübertragungselement distal vor dem ersten Verbindungselement übergreift und eine Breite aufweist, die kleiner als der maximale Querschnitt des ersten Verbindungselements ist.

Ebenso bevorzugt und entsprechend weist die zweite Aufnahme einen Aufnahmeabschnitt, in dem das zweite Verbindungselement im kraftschlüssig verbundenen Zustand zu liegen kommt, und einen Verriegelungsabschnitt auf, der einen Schlitz aufweist, der das Kraftübertragungselement distal vor dem zweiten Verbindungselement übergreift und eine Breite aufweist, die kleiner als der maximale Querschnitt des zweiten Verbindungselements ist.

Diese vorstehend genannten Ausgestaltungen sind insbesondere mit der drehbaren Ausgestaltung des zuvor genannten Kupplungselements von Vorteil, weil sich die kraftschlüssige Verbindung zwischen dem ersten bzw. zweiten Kraftübertragungselement und der ersten bzw. zweiten Aufnahme sehr einfach gestaltet, indem nämlich das erste bzw. zweite Verbindungselement zunächst in der Einführstellung des Kupplungselements in den Aufnahmeabschnitt eingeführt werden kann, wonach das Kupplungselement um eine Drehung, beispielsweise um 90°, um eine senkrecht zur Längsrichtung des jeweiligen Kraftübertragungselements verlaufenden Drehachse gedreht wird, wodurch die Ränder des jeweiligen Schlitzes das jeweilige Kraftübertragungselement distal vor dessen Verbindungselement übergreifen, um eine besonders stabile und möglichst spielfreie kraftschlüssige Verbindung des jeweiligen Kraftübertragungselements in der jeweiligen Aufnahme herzustellen.

In einer weiteren bevorzugten Ausgestaltung des Zangensystems umfasst dieses zumindest einen zweiten Griff, der zumindest ein bewegliches Griffteil aufweist, wobei der zumindest eine zweite Griff eine zweite Kupplungsvorrichtung aufweist, mittels der nur das zweite Kraftübertragungselement kraftschlüssig mit dem beweglichen Griffteil verbunden werden kann, während eine kraftschlüssige Verbindung mit dem ersten Kraftübertragungselement ausgeschlossen ist.

Diese Ausgestaltung hat nun den besonderen Vorteil, dass das Zangensystem einerseits eine Art Griffe aufweist, die es erlauben, mit demselben Griff unterschiedlich dimensionierte bzw. für unterschiedliche Kraftübertragung ausgelegte Kraftübertragungselemente mit demselben Griff zu verbinden, wobei diese Griffe hinsichtlich ihrer maximalen Krafterzeugung so ausgelegt sind, dass auch das schwächer dimensionierte Kraftübertragungselement nicht einer Bruchgefahr unterliegt, während das Zangensystem zumindest eine zweite Art von Griffen umfasst, die für eine höhere Krafterzeugung ausgelegt sind, während mit dieser Art Griffen ausschließlich entsprechend stärker dimensionierte Kraftübertragungselemente kraftschlüssig verbunden werden können. In dieser Ausgestaltung werden somit die Vorteile der oben genannten Codierung von Kraftübertragungselementen auf bestimmte Griffe und zum anderen die Anzahl an Kombiniermöglichkeiten verschiedener Kraftübertragungselemente mit ein und demselben Griff in einem Zangensystem miteinander kombiniert.

In diesem Zusammenhang ist es weiter bevorzugt, wenn die zweite Kupplungsvorrichtung eine Aufnahme für das zweite Verbindungselement aufweist, die so ausgebildet ist, dass das erste Verbindungselement in der Aufnahme nicht verriegelt werden kann.

Diese Maßnahme hat den Vorteil, dass bereits bei der ersten Betätigung des Griffs für die Bedienungsperson erkennbar keine Kraftübertragung vom Griff auf das Werkzeug vorhanden ist, so dass ein Fehlgebrauch von vornherein ausgeschlossen ist.

Dabei ist es weiterhin bevorzugt, wenn die Aufnahme der zweiten Kupplungsvorrichtung gleich oder im Wesentlichen gleich zu der zweiten Aufnahme der ersten Kupplungsvorrichtung ausgebildet ist.

Beispielsweise kann die zweite Kupplungsvorrichtung ebenfalls als drehbares Kupplungselement ausgebildet sein, bei dem dann im Unterschied zu dem Kupplungselement der ersten Kupplungsvorrichtung nur die zweite Aufnahme vorhanden ist, während die erste Aufnahme bei diesem Kupplungselement fehlt. Der Vorteil dieser Maßnahme besteht darin, dass für das gesamte Zangensystem im Wesentlichen gleiche Teile verwendet werden können, wobei nur geringfügige Modifikationen für die unterschiedlichen Funktionen vorgenommen werden müssen, was die Herstellungskosten des erfindungsgemäßen Zangensystems reduziert.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: ein zerlegbares medizinisches Zangensystem, wobei beispielhaft zwei Zangen in zerlegtem Zustand gezeigt sind;
- Fig. 2: eine der beiden Zangen in Fig. 1 in zusammengesetztem Zustand, teilweise im Längsschnitt;
- Fig. 3: die andere der beiden Zangen in Fig. 1 im zusammengesetzten Zustand, teilweise im Längsschnitt;
- Fig. 4a) und b): eine Kupplungsvorrichtung der Zange in Fig. 2 im vergrößerten Maßstab und im Längsschnitt, wobei gemäß Fig. 4a) ein Kraftübertragungselement in die Kupplungsvorrichtung eingeführt und gemäß Fig. 4b) in dieser verriegelt ist;
- Fig. 5a) und b) Fig. 4a) und b): entsprechende Darstellungen der Verbindung eines weiteren Kraftübertragungselements mit der Kupplungsvorrichtung in Fig. 4a) und b);
- Fig. 6: eine Kupplungsvorrichtung der Zange in Fig. 3 im vergrößerten Maßstab und im Längsschnitt, wobei in Fig. 6a) das Kraftübertragungselement gemäß Fig. 4a) in die Kupplungsvorrichtung eingeführt ist, und Fig. 6b) zeigt, dass dieses erste Kraftübertragungselement nicht mit dieser Kupplungsvorrichtung kraftschlüssig verbunden werden kann;
- Fig. 7: ein Kupplungselement der Kupplungsvorrichtung in Fig. 4 und 5 in einer perspektivischen Darstellung und in noch weiter vergrößertem Maßstab; und
- Fig. 8a) bis c): schematische Darstellungen der Kupplungsvorrichtung in Fig. 4, 5 oder 6 in Draufsicht zur Erläuterung eines Steuermechanismus zum Überführen des Kupplungselements von einer Einführstellung gemäß Fig. 8a) in eine Verriegelungsstellung gemäß Fig. 8c).

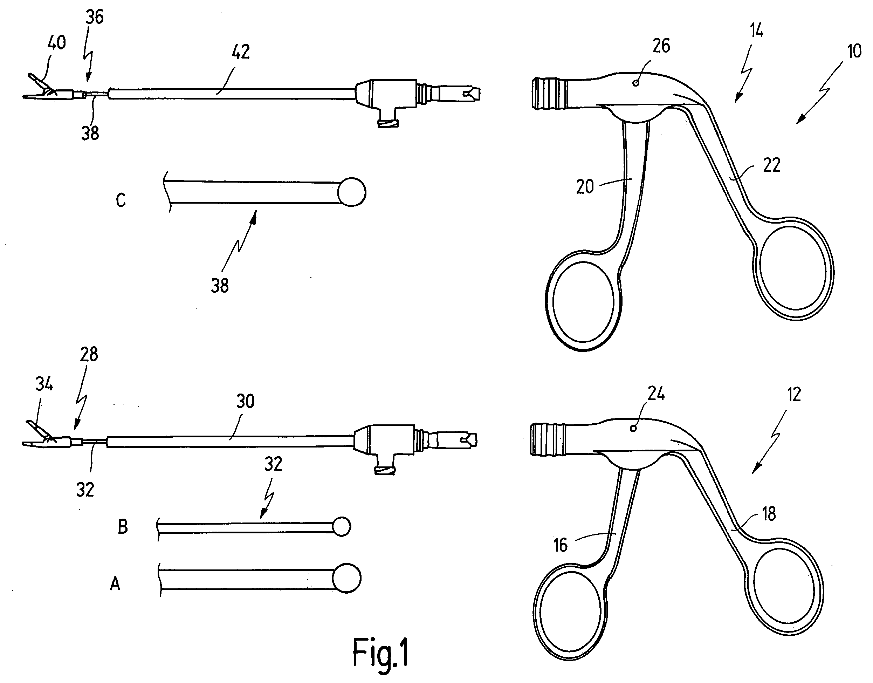

In Fig. 1 ist ein mit dem allgemeinen Bezugszeichen 10 versehenes zerlegbares medizinisches Zangensystem dargestellt. Das Zangensystem 10 wird ohne Beschränkung der Allgemeinheit in der Hals-, Nasen- und Ohrenmedizin bzw. -chirurgie verwendet.

Das Zangensystem 10 weist einen ersten Griff 12 und einen zweiten Griff 14 auf. Der erste Griff 12 weist ein erstes bewegliches Griffteil 16 und ein erstes unbewegliches Griffteil 18 auf. Ebenso weist der zweite Griff 14 ein zweites bewegliches Griffteil 20 und ein zweites unbewegliches Griffteil 22 auf.

Der erste Griff 12 ist zur Erzeugung einer maximalen Kraft ausgelegt, die kleiner ist als die maximale Kraft, die mit dem zweiten Griff 14 erzeugt werden kann. Dies ist in dem gezeigten Ausführungsbeispiel dadurch realisiert, dass das erste bewegliche Griffteil 16 des ersten Griffs 12 in Bezug auf eine erste Schwenkachse 24 eine kleinere Hebellänge besitzt als das zweite bewegliche Griffteil 20 in Bezug auf eine zweite Schwenkachse 26.

In Fig. 1 ist weiterhin beispielhaft ein erster Arbeitseinsatz 28 und ein Schaft 30 gezeigt, wobei die Anordnung aus dem Arbeitseinsatz 28 und dem Schaft 30 mit dem ersten Griff 12 verbunden werden kann, wobei in Fig. 1 der Arbeitseinsatz 28 und der Schaft 30 vom Griff 12 abgenommen sind. Der erste Arbeitseinsatz 28 weist ein erstes Kraftübertragungselement 32 auf, das einerseits distal mit einem beweglichen Werkzeug 34 verbunden ist, das beispielsweise als bewegliches schneidendes, stanzendes oder fassendes Maulteil ausgebildet ist. Das Kraftübertragungselement 32 ist in Form einer Zug-/Druckstange ausgebildet. Ein proximales Ende des Kraftübertragungselements 32, das in Fig. 1 nicht dargestellt ist, dient zur kraftschlüssigen Verbindung mit dem ersten Griff 12, genauer gesagt mit dem ersten beweglichen Griffteil 16, so dass eine Bewegung des ersten beweglichen Griffteils 16 in eine axiale Bewegung des ersten Kraftübertragungselements 32 umgesetzt wird, wobei Letztere das distale Werkzeug 34 bewegt. In Fig. 1 ist der erste Arbeitseinsatz 28 von dem Schaft 30 gelöst und geringfügig nach distal aus dem Schaft 30 herausgezogen dargestellt, so dass das proximale Ende des ersten Kraftübertragungselements 32 in Fig. 1 nicht sichtbar ist.

Weiterhin ist in Fig. 1 ein zweiter Arbeitseinsatz 36 mit einem zweiten Kraftübertragungselement 38, einem zweiten distalen beweglichen Werkzeug 40 und ein zweiter Schaft 42 dargestellt.

Das erste Kraftübertragungselement 32, das mit dem ersten Griff 12 kraftschlüssig verbindbar ist, kann ein solches sein, das für eine Kraftübertragung einer hohen maximalen Kraft ausgelegt ist, wie es schematisch und vergrößert in dem Ausschnitt A in Fig. 1 dargestellt ist, oder ein solches Kraftübertragungselement, das für Kraftübertragung einer niedrigen maximalen Kraft ausgelegt ist, wie es in dem Ausschnitt B in Fig. 1 dargestellt ist. Entsprechend ist das im Ausschnitt B dargestellte Kraftübertragungselement mit geringerer Stärke als das im Ausschnitt A dargestellte Kraftübertragungselement ausgebildet.

Demgegenüber ist der zweite Griff 14, der zur Erzeugung großer Kräfte ausgelegt ist, nur mit einem solchen Kraftübertragungselement 38 kraftschlüssig verbindbar, das entsprechend zur Kraftübertragung hoher Kräfte ausgelegt ist, wie in dem Ausschnitt C in Fig. 1 dargestellt ist, der dem Ausschnitt A in Fig. 1 entspricht.

In der nachfolgenden Beschreibung wird das erste Kraftübertragungselement 32 als ein solches beschrieben, das zur Kraftübertragung einer niedrigen maximalen Kraft ausgelegt ist, und das zweite Kraftübertragungselement 38 als ein solches, das zur Kraftübertragung einer hohen maximalen Kraft ausgelegt ist, die also größer ist als die maximale Kraft, die mit dem ersten Kraftübertragungselement 32 übertragen werden kann.

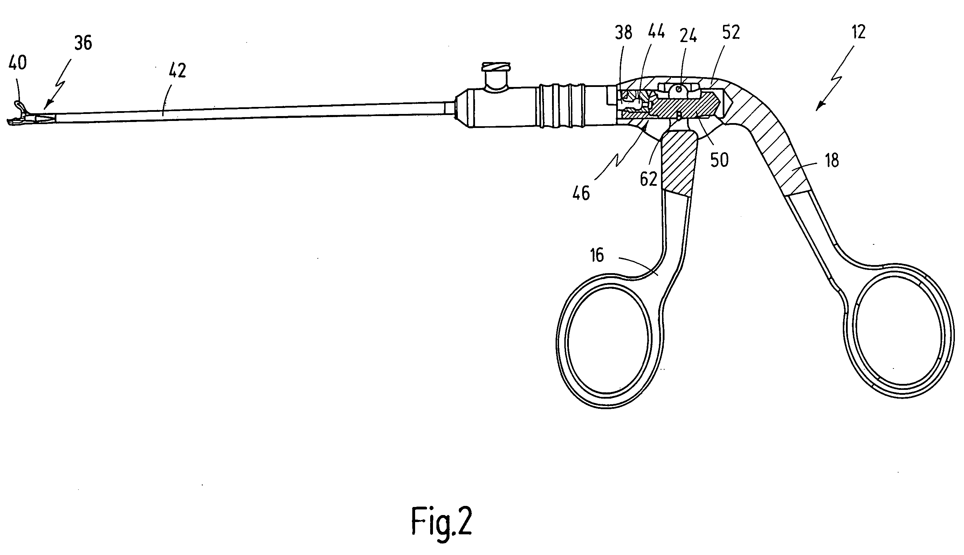

In Fig. 2 ist eine aus dem ersten Griff 12 und dem zweiten Schaft 42 nebst zweitem Arbeitseinsatz 36 und distalem Werkzeug 40 einschließlich Kraftübertragungselement 38 zusammengesetzte Zange dargestellt. Das Kraftübertragungselement 38 weist an seinem proximalen Ende ein zweites Verbindungselement 44 auf, das kugelförmig ausgebildet ist. Gemäß der Schnittdarstellung in Fig. 2 weist der Griff 12 eine Kupplungsvorrichtung 46 zur kraftschlüssigen Verbindung des Kraftübertragungselements 38 mit dem Griff 12, genauer gesagt dem beweglichen Griffteil 16, auf. In dem in Fig. 2 gezeigten Zustand ist das Verbindungselement 44 mit der ersten Kupplungsvorrichtung verriegelt, so dass die in Fig. 2 dargestellte Zange betriebsbereit ist.

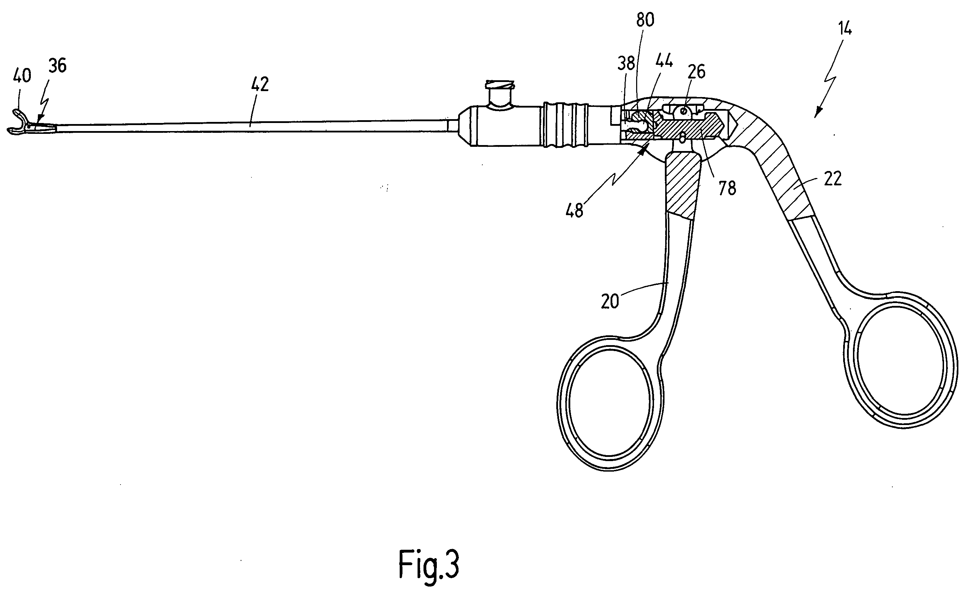

In Fig. 3 ist in gleicher zeichnerischer Darstellung wie in Fig. 2 eine aus dem Griff 14 und dem Arbeitseinsatz 36 mit Schaft 42 und Kraftübertragungselement 38 zusammengesetzte Zange dargestellt, wobei der Griff 14 eine Kupplungsvorrichtung 48 aufweist, die nachfolgend näher beschrieben wird.

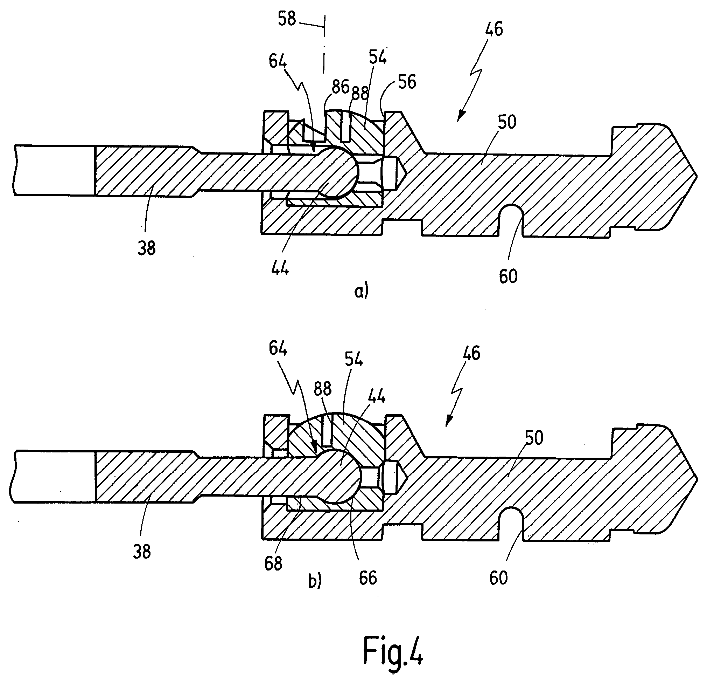

Zunächst wird mit Bezug auf Fig. 2 und 4a) und b) die Kupplungsvorrichtung 46 näher beschrieben.

Die Kupplungsvorrichtung 46 weist einen Schieber 50 auf, der in einem Griffgehäuse 52 des Griffs 12 axial beweglich ist, d.h. in Längsrichtung des Schafts 42 bzw. des Kraftübertragungselements 38. Die Kupplungsvorrichtung 46 weist weiterhin ein Kupplungselement 54 auf, das in einer Sacklochaufnahme 56 des Schiebers 50 angeordnet ist, wobei das erste Kupplungselement 54 relativ zu dem Schieber 50 axial unbeweglich ist, in der Sacklochaufnahme 56 jedoch um eine Achse 58, die senkrecht zur Längsrichtung des Kraftübertragungselements 38 verläuft, drehbeweglich ist.

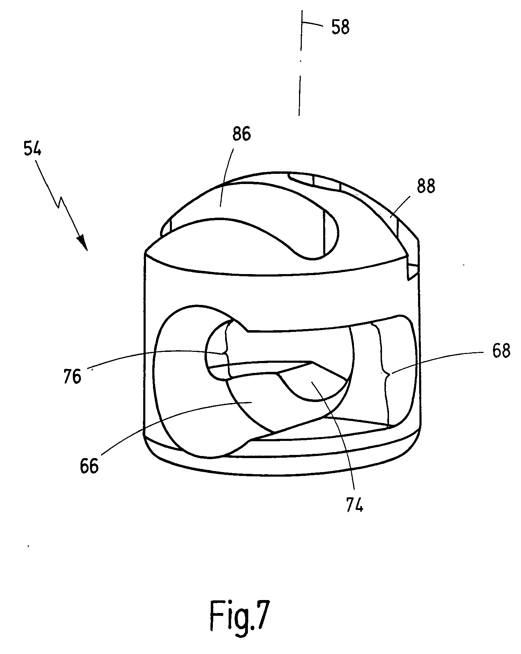

Das erste Kupplungselement 54 ist in Fig. 7 in Alleinstellung und nochmals vergrößert dargestellt.

Der Schieber 50 weist eine Ausnehmung 60 auf, in die ein Mitnehmerstift 62 des ersten beweglichen Griffteils 16 eingreift, so dass bei einer Verschwenkung des ersten beweglichen Griffteils 16 um die Schwenkachse 24 der Schlitten 50 in axialer Richtung nach distal oder proximal, je nach Schwenkrichtung des Griffteils 16, axial bewegt wird.

Das Kupplungselement 54 weist eine als Ausnehmung in dem Kupplungselement 54 ausgebildeten Aufnahme 64 zur kraftschlüssigen Verbindung des Verbindungselements 44 des Kraftübertragungselements 38 auf. Die Aufnahme 64 ist im Querschnitt dazu im Wesentlichen kugelförmig ausgebildet, so dass das kugelförmig ausgebildete Verbindungselement 44 formschlüssig in der Aufnahme 64 aufgenommen werden kann.

Die Aufnahme 64 weist genauer einen Aufnahmeabschnitt 66, in dem das zweite Verbindungselement 44 im kraftschlüssig verbundenen Zustand zu liegen kommt, und einen Verriegelungsabschnitt 68 auf, der einen Schlitz aufweist, der das Kraftübertragungselement 38 distal vor dem Verbindungselement 44 übergreift und eine Breite aufweist, die kleiner als der maximale Querschnitt des Verbindungselements 44 ist, wie aus Fig. 4b) hervorgeht. Wie in Fig. 7 dargestellt ist, erstreckt sich der Verriegelungsabschnitt 68 in Umfangsrichtung um die Drehachse 58 seitlich von dem Aufnahmeabschnitt 66 weg. In dieser Ausgestaltung besteht zwischen dem Verbindungselement 44 und der Aufnahme 64 im verriegelten Zustand ein Formschluss.

Das Kupplungselement 54 ist in der Sacklochaufnahme 56 um etwa 90° - wobei diese Drehung auch einem anderen Verdrehwinkel entsprechen kann - um die Drehachse 58 drehbar, und zwar zwischen einer ersten Stellung (Einführstellung), die in Fig. 4a) gezeigt ist, und einer zweiten Stellung (Verriegelungsstellung), die in Fig. 4b) dargestellt ist. In der in Fig. 4b) dargestellten Stellung des Kupplungselements 54 ist das Kraftübertragungselement 38 kraftschlüssig mit der Kupplungsvorrichtung 46 und damit mit dem Griff 12 bzw. dem beweglichen Griffteil 16 verbunden.

Später wird noch beschrieben, wie die Drehbewegung des Kupplungselements 54 aus einer axialen Bewegung des Schiebers 50 heraus abgeleitet wird.

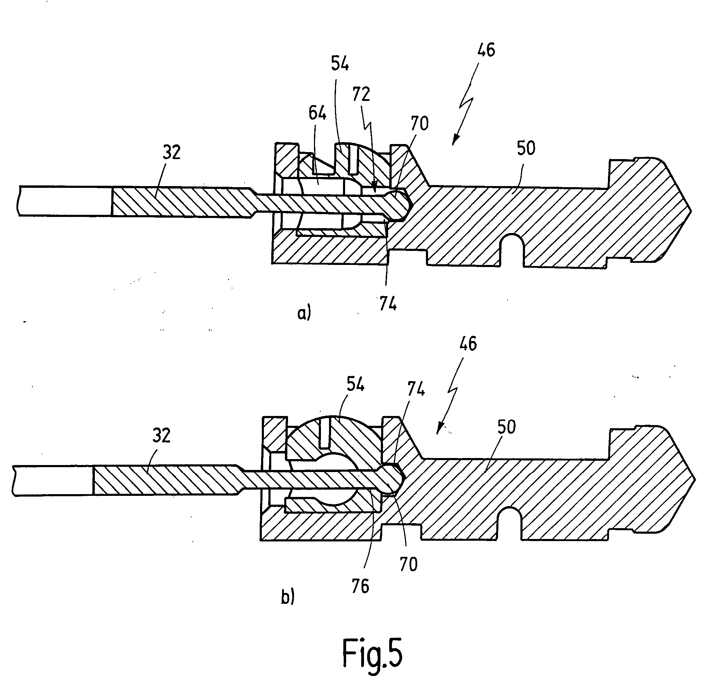

In Fig. 5a) und b) ist nun gezeigt, dass mit der Kupplungsvorrichtung 46 nicht nur das für eine hohe Kraftübertragung ausgelegte Kraftübertragungselement 38 kraftschlüssig verbunden werden kann, sondern auch das für eine Kraftübertragung einer geringeren maximalen Kraft ausgelegte Kraftübertragungselement 32 kraftschlüssig verbunden werden kann.

Das Kraftübertragungselement 32 ist gegenüber dem Kraftübertragungselement 38 insgesamt mit geringerer Stärke ausgebildet und weist an seinem proximalen Ende ein Verbindungselement 70 auf, das zwar ebenfalls wie das zweite Verbindungselement 44 kugelförmig ausgebildet ist, sich jedoch von Letzterem hinsichtlich seiner Querschnittsgröße unterscheidet, d.h. kleiner ist.

Abweichend von dem gezeigten Ausführungsbeispiel ist es jedoch ebenso möglich, dass sich das Verbindungselement 70 und das Verbindungselement 44 nicht oder nicht nur hinsichtlich ihrer Querschnittsgröße unterscheiden, sondern es sind auch Ausgestaltungen möglich, bei denen sich die Verbindungselemente 70 und 44 hinsichtlich ihrer Querschnittsform unterscheiden. Beispielsweise kann das erste Verbindungselement 70 anstatt kugelförmig quaderförmig, zylinderförmig oder als Abflachungen ausgebildet sein, nur um Beispiele zu nennen.

Die Kupplungsvorrichtung 46 weist für das erste Verbindungselement 70 eine Aufnahme 72 auf, die von der Aufnahme 64 axial beabstandet ist, wobei die Aufnahme 72 proximal von der Aufnahme 64 angeordnet ist.

Die Aufnahme 72 weist wiederum einen Aufnahmeabschnitt 74 auf, der in dem gezeigten Ausführungsbeispiel teilweise auch in dem Schlitten 50 ausgebildet ist. Der Aufnahmeabschnitt 74 kann jedoch auch vollständig in dem Kupplungselement 54 angeordnet sein.

Die Aufnahme 72 weist des Weiteren einen Verriegelungsabschnitt 76 auf, der einen Schlitz aufweist, der das Kraftübertragungselement 32 distal vor dem Verbindungselement 70 übergreift und eine Breite aufweist, die kleiner als der maximale Querschnitt des Verbindungselements 70 ist. Gemäß Fig. 7 erstreckt sich der Verriegelungsabschnitt 76 in Umfangsrichtung um die Drehachse 58 seitlich von dem Aufnahmeabschnitt 74, und zwar im gleichen Drehsinn wie der Verriegelungsabschnitt 68 der Aufnahme 64. Fig. 5a) zeigt das Kupplungselement 54 wiederum in der Einführstellung, in der das Kraftübertragungselement 32 mit dem Verbindungselement 70 voran in die Aufnahme 72 eingeführt werden kann, bzw. wieder herausgezogen werden kann, und Fig. 5b) zeigt das Kupplungselement 54 in einer um die Drehachse 58 um 90° gegenüber der Einführstellung gedrehten Verriegelungsstellung, in der das Kraftübertragungselement 32 kraftschlüssig mit der Kupplungsvorrichtung 46 verbunden ist.

Das Verbindungselement 70 greift durch die Aufnahme 64 hindurch, wenn es mit der Aufnahme 72 verbunden ist. Dazu ist die Aufnahme 64 entsprechend mit einem geeignet großen Querschnitt ausgebildet. Die Länge der Kraftübertragungselemente 32 und 38 ist vorzugsweise unterschiedlich, so dass sich unabhängig vom dem Kraftübertragungselement 32 oder 38 das Werkzeug 34 oder 40 am distalen Ende im gleichen Abstand vom Griff 12 befindet.

Mit dem Griff 12 kann somit wahlweise das Kraftübertragungselement 32 oder das Kraftübertragungselement 38 kraftschlüssig verbunden werden. Da der Griff 12 nur eine maximale Kraft erzeugen kann, dem das Kraftübertragungselement 32 standhalten kann, besteht keine oder nur eine geringe Bruchgefahr, wenn das "schwächere" Kraftübertragungselement 32 mit dem Griff 12 verbunden wird.

Der Griff 14, der zur Erzeugung höherer Kräfte ausgelegt ist, ist dagegen, wie nachfolgend beschrieben wird, nur mit dem zweiten Kraftübertragungselement 38 kraftschlüssig verbindbar, während das erste Kraftübertragungselement 32 mit dem zweiten Griff 14 nicht kraftschlüssig verbindbar ist.

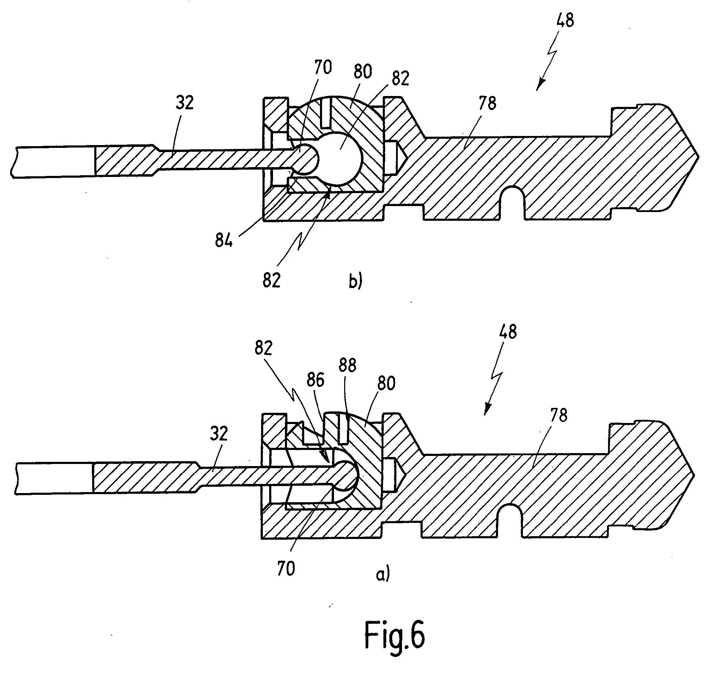

Gemäß Fig. 3 und 6a), b) weist die Kupplungsvorrichtung 48 einen Schieber 78 auf, der mit dem Schieber 50 der Kupplungsvorrichtung 46 vorzugsweise identisch ausgebildet ist, was die Herstellungskosten verringert, weil für den Griff 12 und den Griff 14 zumindest teilweise identische Bauteile verwendet werden können. Auch die Funktion des Schiebers 78 ist identisch mit der Funktion des Schiebers 50, wie sie oben bereits beschrieben wurde.

Die Kupplungsvorrichtung 48 weist ferner ein Kupplungselement 80 auf, das mit dem Kupplungselement 54 bis auf folgende Ausnahme ebenfalls identisch ausgebildet ist. Im Unterschied zu dem Kupplungselement 54 weist das Kupplungselement 80 nämlich nur eine Aufnahme 82 auf, die identisch mit der Aufnahme 64 des Kupplungselements 54 ausgebildet ist. Dagegen fehlt bei dem Kupplungselement 80 die Aufnahme 72 des Kupplungselements 54. Dies hat zur Folge, dass sich das Verbindungselement 70 des Kraftübertragungselements 32 in der in Fig. 6a) gezeigten Einführstellung des Kupplungselements 80 zwar in die Aufnahme 82 einführen lässt, jedoch wird zwischen der Aufnahme 82 und dem Verbindungselement 70 bei einer Drehung des Kupplungselements 80 in seiner Verriegelungsstellung gemäß Fig. 6b) keine kraftschlüssige Verbindung des Verbindungselements 70 mit der Aufnahme 82 hergestellt, weil das Verbindungselement 70 einen kleineren Querschnitt aufweist als der Verriegelungsabschnitt 84 der Aufnahme 82.

Somit kann es nicht vorkommen, dass eine Bedienungsperson das schwächer dimensionierte Kraftübertragungselement 32 mit dem größer dimensionierten zweiten Griff 14 verbinden kann, wodurch ein Bruch des Kraftübertragungselements 32 in jedem Fall ausgeschlossen wird. Der Griff 14 besitzt somit eine Codierungseigenschaft, die nur eine kraftschlüssige Verbindung des stärker dimensionierten Kraftübertragungselements 38 mit dem Griff 14, nicht jedoch eine kraftschlüssige Verbindung des schwächer dimensionierten Kraftübertragungselements 32 mit dem Griff 14 zulässt.

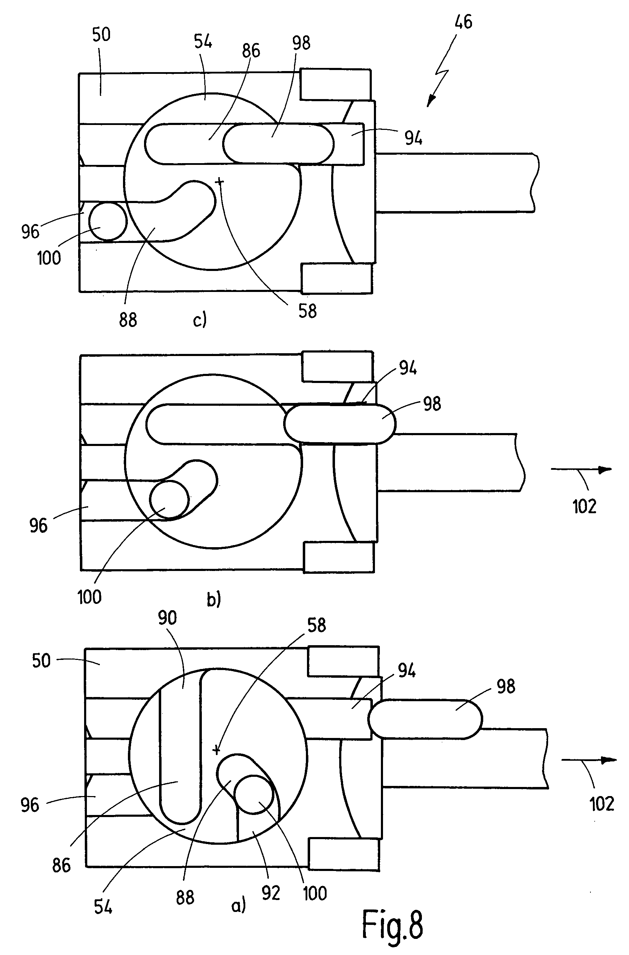

Sowohl das Kupplungselement 54 als auch das Kupplungselement 80 leiten ihre Drehbewegung zwischen der Einführstellung und der Verriegelungsstellung aus einer axialen Bewegung des Schiebers 50 bzw. 78 ab, wie hiernach anhand der schematischen Zeichnung in Fig. 8a) bis c) in Bezug auf die Kupplungsvorrichtung 46 des Griffs 12 beschrieben wird. Fig. 8a) bis c) sind Draufsichten auf den ausschnittsweise dargestellten Schieber 50 und das Kupplungselement 54.

Das Kupplungselement 54 weist eine erste Steuerkurve 86 auf, die in Form einer langlochförmigen Ausnahme ausgebildet ist, die auch in Fig. 7 zu sehen ist. Das Kupplungselement 54 weist weiterhin eine zweite Steuerkurve 88 auf, die ebenfalls als langlochförmige Ausnehmung ausgebildet ist, im Unterschied zu der ersten Steuerkurve 86 jedoch einen gekrümmten Verlauf einnimmt. Die erste Steuerkurve 86 ist an einem Ende 90 und die zweite Steuerkurve 88 an einem Ende 92 offen, wobei die Enden 90 und 92 bezüglich der Drehachse 58, die in Fig. 8a) bis c) senkrecht zur Zeichenebene verläuft, um etwa 180° versetzt sind.

Der Schieber 50 weist zwei Ausnehmungen 94 und 96 auf.

Zur Steuerung der Drehbewegung des Kupplungselements 54 aus der axialen Bewegung des Schiebers 50 heraus sind zwei Führungselemente 98 und 100 vorgesehen, die unbeweglich sind und beispielsweise an der Innenseite des Griffgehäuses 52 befestigt sind.

Fig. 8a) zeigt das Kupplungselement 54 in seiner Einführstellung, in der das Kraftübertragungselement 32 oder das Kraftübertragungselement 38 mit ihrem jeweiligen Verbindungselement 44 oder 70 voran in die jeweilige oben beschriebene erste oder zweite Aufnahme eingeführt werden können. Das Führungselement 100 steht in dieser Stellung mit der zweiten Steuerkurve 88 in Eingriff.

Wird der Schieber 50 ausgehend von Fig. 8a) in Richtung eines Pfeils 102 axial bewegt, bewirkt das Führungselement 100, das in die zweite Steuerkurve 88 eingreift, aufgrund der gekrümmten Ausgestaltung der zweiten Steuerkurve 88 eine 90°-Drehung des Kupplungselements 54 relativ zu dem Schieber 50. Währenddessen fährt das Führungselement 98 zunächst in die Ausnehmung 94 des Schiebers 50 ein.

Bei einer weiteren axialen Bewegung des Schiebers 50 in Richtung des Pfeils 102 tritt nun das Führungselement 100 aus der zweiten Steuerkurve 88 aus, und das Führungselement 98 tritt teilweise in die erste Steuerkurve 86 ein, verbleibt aber auch zum Teil in der Ausnehmung 94 des Schiebers 50, wodurch das Kupplungselement 54 drehfest mit dem Schieber 50 verriegelt ist. Nunmehr ist das Kraftübertragungselement 32 oder 38 kraftschlüssig mit der Kupplungsvorrichtung 46 verbunden. Die Zerlegung erfolgt entsprechend auf umgekehrte Weise.

Die axiale Beweglichkeit des Schiebers 50 ist im einsatzbereiten Betriebszustand der oben beschriebenen Zange durch einen Anschlag begrenzt, der durch eine proximale Schaftkante gebildet wird. Erst durch Lösen beispielsweise des Schafts 42 von dem Handgriff 12 (vgl. Fig. 1 und Fig. 2) kann der Schieber 50 über seinen dem Arbeitsbereich entsprechenden axialen Bewegungshub, der zum Betätigen bspw. des Werkzeugs 34 erforderlich ist, hinaus axial bewegt werden, um die Verbindung bzw.

Trennung des Kraftübertragungselements 32 oder 38 mit bzw. von der Kupplungsvorrichtung 46 zu ermöglichen.

## Patentansprüche

1. Zerlegbares medizinisches Zangensystem, mit einem ersten Kraftübertragungselement (32), das zur Kraftübertragung einer ersten maximalen Kraft ausgelegt ist, mit zumindest einem zweiten Kraftübertragungselement (38), das zur Kraftübertragung einer zweiten maximalen Kraft ausgelegt ist, die größer ist als die erste maximale Kraft, und mit zumindest einem ersten Griff (12), der zumindest ein bewegliches Griffteil (16) aufweist, wobei der zumindest eine erste Griff (12) eine Kupplungsvorrichtung (46) aufweist, mittels der eines der Kraftübertragungselemente (32, 38) kraftschlüssig mit dem beweglichen Griffteil (16) verbunden werden kann, wobei das erste Kraftübertragungselement (32) ein erstes Verbindungselement (70) und das zumindest zweite Kraftübertragungselement (38) ein zweites Verbindungselement (44) zur jeweiligen Verbindung mit der Kupplungsvorrichtung (46) aufweist, wobei die Kupplungsvorrichtung (46) eine erste Aufnahme (72), die zur kraftschlüssigen Verbindung mit dem ersten Verbindungselement (32) ausgelegt ist, und zumindest eine zweite Aufnahme (64) aufweist, die zur kraftschlüssigen Verbindung mit dem zweiten Verbindungselement (44) ausgelegt ist, **dadurch gekennzeichnet, dass** sich die Querschnittsgröße und/oder Querschnittsform des ersten Verbindungselements (70) quer zur Längsrichtung des ersten Kraftübertragungselements (32) von der Querschnittsgröße und/oder Querschnittsform des zweiten Verbindungselements (44) quer zur Längsrichtung des zweiten Kraftübertragungselements (38) unterscheidet.

2. Zangensystem nach Anspruch 1, **dadurch gekennzeichnet, dass** die erste Aufnahme (72) von der zweiten Aufnahme (64) axial beabstandet ist, wobei vorzugsweise das erste Verbindungselement (70) durch die zweite Aufnahme (64) hindurchgreift, wenn es mit der ersten Aufnahme (72) kraftschlüssig verbunden ist.

3. Zangensystem nach Anspruch 1 oder 2, **dadurch gekennzeichnet, dass** die Kupplungsvorrichtung (46) ein zwischen einer ersten Stellung und einer zweiten Stellung bewegliches Kupplungselement (54) aufweist, das die erste und die zumindest zweite Aufnahme (64, 72) zumindest teilweise aufweist, wobei in der ersten Stellung das erste oder das zumindest zweite Verbindungselement (44, 70) in die erste oder zweite Aufnahme (64, 72) einführbar oder herausnehmbar ist, und in der zweiten Stellung das erste oder das zumindest zweite Verbindungselement (44, 70) in der ersten bzw. zweiten Aufnahme (64, 72) verriegelt ist.

4. Zangensystem nach Anspruch 3, **dadurch gekennzeichnet, dass** das Kupplungselement (54) durch eine Drehbewegung von der ersten Stellung in die zweite Stellung bzw. umgekehrt bewegbar ist, dass das Kupplungselement (54) mit einem axial beweglichen Schieber (50), der mit dem beweglichen Griffteil (16) verbunden ist, verbunden ist und relativ zu dem Schieber (50) axial unbeweglich ist, und dass die Drehbewegung aus einer axialen Bewegung des Schiebers (50) abgeleitet wird.

5. Zangensystem nach einem der Ansprüche 1 bis 4, **dadurch gekennzeichnet, dass** die erste Aufnahme (72) einen Aufnahmeabschnitt (74), in dem das erste Verbindungselement (70) im kraftschlüssig verbundenen Zustand zu liegen kommt, und einen Verriegelungsabschnitt (76) aufweist, der einen Schlitz aufweist, der das Kraftübertragungselement (32) distal vor dem ersten Verbindungselement (70) übergreift und eine Breite aufweist, die kleiner als der maximale Querschnitt des ersten Verbindungselements (70) ist, und/oder dass die zweite Aufnahme (64) einen Aufnahmeabschnitt (66), in dem das zweite Verbindungselement (44) im kraftschlüssig verbundenen Zustand zu liegen kommt, und einen Verriegelungsabschnitt (68) aufweist, der einen Schlitz aufweist, der das Kraftübertragungselement (38) distal vor dem zweiten Verbindungselement (44) übergreift und eine Breite aufweist, die kleiner als der maximale Querschnitt des zweiten Verbindungselements (44) ist.

6. Zangensystem nach einem der Ansprüche 1 bis 5, mit zumindest einem zweiten Griff (14), der zumindest ein bewegliches Griffteil (20) aufweist, **dadurch gekennzeichnet, dass** der zumindest eine zweite Griff (14) eine zweite Kupplungsvorrichtung (48) aufweist, mittels der nur das zweite Kraftübertragungselement (38) kraftschlüssig mit dem beweglichen Griffteil (20) verbunden werden kann, während eine kraftschlüssige Verbindung mit dem ersten Kraftübertragungselement (32) ausgeschlossen ist.

7. Zangensystem nach Anspruch 6, **dadurch gekennzeichnet, dass** die zweite Kupplungsvorrichtung (48) eine Aufnahme (82) für das zweite Verbindungselement (44) aufweist, die so ausgebildet ist, dass das erste Verbindungselement (70) in der Aufnahme (82) nicht verriegelt werden kann.

8. Zangensystem nach Anspruch 6 oder 7, **dadurch gekennzeichnet, dass** die Aufnahme (82) der zweiten Kupplungsvorrichtung (48) gleich oder im wesentlichen gleich zu der zweiten Aufnahme (64) der ersten Kupplungsvorrichtung (46) ausgebildet ist.

9. Zangensystem nach einem der Ansprüche 1 bis 8, **dadurch gekennzeichnet, dass** das erste Verbindungselement (70) kugelförmig mit einem ersten Durchmesser und das zweite Verbindungselement (44) kugelförmig mit einem zweiten Durchmesser ist, der größer als der erste Durchmesser ist.

## Claims

1. A dismantlable medical forceps system, comprising a first force transmission element (32), which is designed to transmit a first maximum force, further comprising at least a second force transmission element (38), which is designed to transmit a second maximum force greater than the first maximum force, and further comprising at least a first grip (12), which has at least one movable grip part (16), the at least one first grip (12) having a coupling device (46) by means of which one of the force transmission elements (32, 38) can be connected with a force fit to the movable grip part (16), the first force transmission element (32) having a first connection element (70) and the at least second force transmission element (38) having a second connection element (44) for respective connection to the coupling device (46), wherein the coupling device (46) has a first seat (72), which is designed for force-fit connection to the first connection element (32), and at least a second seat (64), which is designed for force-fit connection to the second connection element (44), **characterized in that** the cross-sectional size and/or the cross-sectional shape of the first connection element (70) transverse to the longitudinal direction of the first force transmission element (32) differs from the cross-sectional size and/or the cross-sectional shape of the second connection element (44) transverse to the longitudinal direction of the second force transmission element (38).

2. The forceps system of Claim 1, **characterized in that** the first seat (72) is at an axial distance from the second seat (64), wherein, preferably, the first connection element (70) engages through the second seat (64) when it is connected with a force fit to the first seat (72).

3. The forceps system of Claim 1 or 2, **characterized in that** the coupling device (46) has a coupling element (54) which can move between a first position and a second position and which at least partially comprises the first and the at least second seats (64, 72), the first or the at least second connection element (44, 70) being able to be introduced into or removed from the first or second seat (64, 72) in the first position, and the first or the at least second connection element (44, 70) being able to be locked in the first or second seat (64, 72) in the second position.

4. The forceps system of Claim 3, **characterized in that** the coupling element (54) can be moved by a rotation movement from the first position to the second position and vice versa, **in that** the coupling element (54) is connected to an axially movable slide (50), which is connected to the movable grip part (16), and is axially immovable relative to the slide (50), and **in that** the rotation movement is derived from an axial movement of the slide (50).

5. The forceps system of anyone of Claims 1 through 4, **characterized in that** the first seat (72) has a seat portion (74), in which the first connection element (70) comes to lie when connected with a force fit, and a locking portion (76) having a slit which engages over the force transmission element (32) distally in front of the first connection element (70) and has a width smaller than the maximum cross section of the first connection element (70), and/or that the second seat (64) has a seat portion (66), in which the second connection element (44) comes to lie when connected with a force fit, and a locking portion (68) having a slit which engages over the force transmission element (38) distally in front of the second connection element (44) and has a width smaller than the maximum cross section of the second connection element (44).

6. The forceps system of anyone of Claims 1 through 5, comprising at least one second grip (14), which has at least one movable grip part (20), **characterized in that** the at least one second grip (14) has a second coupling device (48) by means of which only the second force transmission element (38) can be connected with a force fit to the movable grip part (20), while a force-fit connection to the first force transmission element (32) is excluded.

7. The forceps system of Claim 6, **characterized in that** the second coupling device (48) has a seat (82) for the second connection element (44), said seat (82) being designed such that the first connection element (70) cannot be locked in the seat (82).

8. The forceps system of Claim 6 or 7, **characterized in that** the seat (82) of the second coupling device (48) is designed identically or substantially identically to the second seat (64) of the first coupling device (46).

9. The forceps system of anyone of Claims 1 through 8, **characterized in that** the first connection element (70) is spherical with a first diameter, and the second connection element (44) is spherical with a second diameter greater than the first diameter.

## Revendications

1. Système de pinces médicales démontables, comprenant un premier élément de transmission de force (32), qui est configuré afin de transmettre une première force maximale, au moins un deuxième élément de transmission de force (38), qui est configuré afn de transmettre une deuxième force maximale, qui est plus importante que la première force maximale, et au moins une première poignée (12), qui présente au moins une partie de poignée (16) mobile, dans lequel l'au moins une première poignée (12) présente un dispositif de couplage (46), au moyen duquel un des éléments de transmission de force (32, 38) peut être relié à force à la partie de poignée (16) mobile, dans lequel le premier élément de transmission de force (32) présente un premier élément de liaison (70) et que l'au moins un deuxième élément de transmission de force (38) présente un deuxième élément de liaison (44) destiné à être relié respectivement au dispositif de couplage (46), dans lequel le dispositif de couplage (46) présente un premier logement (72), qui est configuré afin d'être relié à force au premier élément de liaison (32), et au moins un deuxième logement (64), qui est configuré afin d'être relié à force au deuxième élément de liaison (44), **caractérisé en ce que** fa dimension et/ou la forme de la section transversale du premier élément de liaison (70) par rapport à la direction longitudinale du premier élément de transmission de force (32) sont différentes de la dimension et/ou de la forme de la section transversale du deuxième élément de liaison (44) de manière transversale par rapport à la direction longitudinale du deuxième élément de transmission (38).

2. Système de pinces selon la revendication 1, **caractérisé en ce que** le premier logement (72) est tenu à distance de manière axiale du deuxième logement (64), dans lequel de préférence le premier élément de liaison (70) vient en prise à travers le deuxième logement (64) lorsqu'il est relié à force au premier logement (72).

3. Système de pinces selon la revendication 1 ou 2, **caractérisé en ce que** le dispositif de couplage (46) présente un élément de couplage (54) mobile entre une première position et une deuxième position, lequel présente le premier et l'au moins un deuxième logement (64, 72), dans lequel dans la première position le premier élément de liaison ou l'au moins un deuxième élément de liaison (44, 70) peut être introduit dans le premier ou le deuxième logement (64, 72) ou en être retiré et que, dans la deuxième position, le premier ou l'au moins un deuxième élément de liaison (44, 70) est verrouillé dans le premier ou le deuxième logement (64, 72).

4. Système de pinces selon la revendication 3, **caractérisé en ce que** l'élément de couplage (54) peut être déplacé par un déplacement en rotation depuis la première position dans la deuxième position, ou inversement, **en ce que** l'élément de couplage (54) est relié à un coulisseau (50) mobile axialement, lequel est relié à la partie de poignée (16) mobile, et n'est pas mobile axialement par rapport au coulisseau (50), et **en ce que** le déplacement en rotation est dérivé d'un déplacement axial du coulisseau (50).

5. Système de pinces selon l'une quelconque des revendications 1 à 4, **caractérisé en ce que** le premier logement (72) présente une section de logement (74), dans laquelle le premier élément de liaison (70) est placé dans l'état relié à force, et une section de verrouillage (76), qui présente une fente, qui surmonte l'élément de transmission de force (32) en dépassant distalement du premier élément de liaison (70) et présente une largeur, qui est inférieure à la section transversale maximale du premier élément de liaison (70), et/ou **en ce que** le deuxième logement (64) présente une section de logement (66), dans laquelle le deuxième élément de liaison (44) est placé dans un état relié à force, et une section de verrouillage (68), qui présente une fente, qui surmonte l'élément de transmission de force (38) en dépassant distalement du deuxième élément de liaison (44) et présente une largeur, qui est inférieure à la section transversale maximale du deuxième élément de liaison (44).

6. Système de pinces selon l'une quelconque des revendications 1 à 5, comprenant au moins une deuxième poignée (14), qui présente au moins une partie de poignée (20) mobile, **caractérisé en ce que** l'au moins une deuxième poignée (14) présente un deuxième dispositif de couplage (48), au moyen duquel seulement le deuxième élément de transmission de force (38) peut être relié à force à la partie de poignée (20) mobile, tandis qu'une liaison à force avec le premier élément de transmission de force (32) est exclue.

7. Système de pinces selon la revendication 6, **caractérisé en ce que** le deuxième dispositif de couplage (48) présente un logement (82) pour le deuxième élément de liaison (44), qui est réalisé de telle manière que le premier élément de liaison (70) ne peut pas être verrouillé dans le logement (82).

8. Système de pinces selon la revendication 6 ou 7, **caractérisé en ce que** le logement (82) du deuxième dispositif de couplage (48) est réalisé de manière identique ou de manière sensiblement identique par rapport au deuxième logement (64) du premier dispositif de couplage (46).

9. Système de pinces selon l'une quelconque des revendications 1 à 8, **caractérisé en ce que** le premier élément de liaison (70) présente une forme sphérique ayant un premier diamètre, et **en ce que** le deuxième élément de liaison (44) présente une forme sphérique ayant un deuxième diamètre, qui est plus grand que le premier diamètre.
